(19) Europäisches Patentamt
European Patent Office
Office européen des brevets

(11) **EP 3 300 669 A1**

(12) **EUROPEAN PATENT APPLICATION**

(43) Date of publication:
**04.04.2018 Bulletin 2018/14**

(51) Int Cl.:
***A61B 17/00*** (2006.01)

(21) Application number: **17154743.3**

(22) Date of filing: **06.02.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **29.09.2016 EP 16191271**

(71) Applicant: **Universität Zürich**
**8006 Zürich (CH)**

(72) Inventors:
• **Devaud, Yannick**
**1700 Fribourg (CH)**
• **Milleret, Vincent**
**8057 Zurich (CH)**
• **Zimmermann, Roland**
**8044 Gockhausen (CH)**
• **Ehrbar, Martin**
**9500 Wil (CH)**
• **Ochsenbein, Nicole**
**8049 Zurich (CH)**

(74) Representative: **Richter, Allen**
**Patentanwalt Dr. Kasche**
**Resirain 1**
**8125 Zollikerberg/Zürich (CH)**

(54) **DEVICE AND METHOD FOR SEALING A MEMBRANE**

(57)     Disclosed embodiments relate to a membrane closure device (100) for closing a perforation in a membrane (10), comprising: a patch guiding mechanism (110) for guiding at least one expandable patch (120) to a perforation site (12) of a membrane (10); and a fastener delivery mechanism (130, 330) for delivering a fastener (140) to the perforation site (12) and for fastening the at least one expandable patch by the fastener (140) to the membrane (10) to seal the perforation in the membrane (10) by the at least one expandable patch (120).

FIG. 1

**Description**

**TECHNICAL FIELD AND BACKGROUND**

Embodiments disclosed herein relate in general to devices and methods for sealing a membrane such as a fetal membrane.

The fetal membrane is a relatively thin tissue layer having a thickness of a few hundred microns that surrounds the fetus during development. The fetal membrane holds amniotic fluid and creates a physical barrier, for example, to protect the fetus from infections and to provide paracrine signalling between the mother and the embryo. Intact fetal membranes are of central importance to a pregnancy. Preterm pre-labour rupture of fetal membranes (PPROM) initiates delivery in most cases, which is a serious complication in early pregnancy. Indeed, preterm birth carries a big risk of complication ranging from incurable diseases decreasing life quality and/or expectancy and range from cerebral palsy, impaired cognitive skills, vision problems, hearing problems, behavioral, psychological as well as chronic health issues to morbidity.

With advances in fetal diagnosis and therapies, fetoscopy has become a clinical routine. This surgical technique aims at treating potentially life-threatening diseases during pregnancy by invasive intervention into the amniotic cavity. Example medical indications include twin-to-twin transfusion syndrome (TTTS), discordant monochorionic twins with a lethal anomaly, reverse arterial perfusion (TRAP) as well as severe congenital diaphragmatic hernia (CDH). Depending on the type of intervention, the injury or perforation created by the fetoscopy can induce iatrogenic PPROM (iPPROM) in 6 to 45% of the cases. This unsolved clinical problem compromises benefits of the surgical intervention and blocks tremendously the advances in treatment technologies.

Since fetal membranes have a limited spontaneous healing potential, the application of natural and synthetic materials to close the defect has been studied in the past. Such approaches have failed to prove clinical relevance due to inadequate material stability or to the absence of healing-inducing factors.

The description above is presented as a general overview of related art in this field and should not be construed as an admission that any of the information it contains constitutes prior art against the present patent application.

**GENERAL DESCRIPTION OF THE INVENTION**

Embodiments of the present invention are aimed at overcoming some of the disadvantages associated with the application of, e.g., polymeric biomimetic gluing material such as "mussel glue" for the sealing of fetal membranes. The potential in the use of such polymeric biomimetic gluing material for the sealing of membranes was described in Lee et al., Nature 2007, 448. 338-41, and further in Bilic et al., American Journal of Obstetrics and Gynecology 2010, 202. 85 e1-9. Further, Haller et al. disclose in Acta biomaterialia 2012, 8. 4365-70 that mussel glue is non-toxic, tightly adheres to fetal membranes and withstands substantial deformation and/or pressure of fetal membranes.

However, some of the problems that were encountered in all the above-noted studies was the difficulty to deliver the gluing material to the desired location and the lack of a reproducible and robust method to apply the gluing material at the desired location for closing the perforated membrane.

Devices and methods according to embodiments of the present invention may overcome at least some of the above-noted problems.

The following description of devices and methods of the invention is given with reference to particular examples, with the understanding that such devices and methods are not limited to these examples.

The disclosed embodiments relate to membrane closure devices and methods for repairing a perforated (also: ruptured) membrane. Such membrane can, for example, include a fetal membrane, vascular membrane, cardiovascular membrane and/or a tympanic membrane. Non-medical applications may for example include the sealing of membranes in industrial applications or environments such as automotive, process engineering, construction, precision mechanics, and/or the like. Correspondingly, while embodiments disclosed herein relate to repairing living tissue membrane, this should by no means be construed limiting. Accordingly, the devices and methods described herein may be applicable to any type of perforated membrane or layer, whether living or non-living.

According to some embodiments, the membrane closure device for closing a perforation in a membrane, comprises a patch delivery mechanism for delivering and deploying one or more (e.g., expandable) patches to a perforation site of the membrane. The membrane closure device further comprises a fastener delivery mechanism for delivering a fastener to the perforation site for fastening the one or more expandable patches, by the fastener, to the membrane to seal the perforation in the membrane. Such fastener can be, for example, a sealant, a sealant-inducing material, healing-inducing matter, a plug, and/or a weld. The thickness of the patch can range, for example, from about 50 micron to about 1mm.

Merely to simplify the discussion that follows, without be construed limiting, the terms "fastener", "glue", and "sealant" may herein be used interchangeably.

The membrane closure device comprises a tube-shaped body (also: tube) having a distal and a proximal end. The tube-shaped body defines internal to the tube a patch delivery passageway (also: operating channel) for delivering the expandable patch in a narrow (e.g., crimped or folded) configuration, via the tube, from the proximal to the distal end for attaching the expandable patch, using the fastener, in an expanded configuration to a surface (e.g., distal surface) of the membrane.

The relative positional term "proximal" as used herein refers to a location that is situated closer from a user of the device during normal use of the device than a location that is "distal" to the user during such normal use. For example, a proximal end is an end point of the tube situated closest to the user of the device, and a distal end is an endpoint situated farthest from the user.

An outer membrane surface of a membrane is a surface that has to be engaged first by the device for gaining access to a cavity enclosed by the membrane. An inner membrane surface may refer to the surface that is in direct contact with the content of the cavity and that can be engaged only after engagement of the device with the outer surface.

Merely to simplify the discussion that follows, the term "proximal membrane surface" may herein also be referred to as "outer surface" or "upper surface". Correspondingly, the term "inner membrane surface" may herein also be referred to as "inner surface" or "undersurface".

In the context of the present disclosure, a longitudinal direction of the tube extends from its proximal end along a longitudinal axis to the distal end. The latitudinal direction is perpendicular to the longitudinal axis, along a latitudinal axis of the tube.

When in the folded configuration, the patch can slide (e.g., by applying a pushing and/or pulling force) through the tube along its longitudinal axis from the distal to the proximal end. Upon exiting the tube's distal end, the patch is set in the expanded configuration (e.g., by expanding in latitudinal direction) for covering the perforation.

The term "user" as used herein may include, for example, a physician or medical doctor. The term "patient" as used herein refers to the person that is subjected to procedures for closing a perforated membrane in the body of the patient.

The terms cited above denote also grammatical variations thereof.

Merely, for brevity and clarity, and therefore without be construed limiting, in the present disclosure a membrane closure device is herein exemplified for use during a minimally invasive surgery (MIS).

The outer diameter of the portions of the tube-shaped body perforating the membrane for, e.g., fetoscopy - which may be performed at a gestational stage ranging, e.g., from week 17 to week 27 - may range, for example, from 1.7 mm to 4 mm. The diameter may depend, inter alia, on the fetoscopy procedure required to be performed. Fetoscopy procedures can include, for example, placental laser surgery and/or shunting. The patch in the folded configuration is configured to slidably fit into the patch delivery passageway, which is internal to the tube-shaped body. In the expanded configuration, the patch's diameter can for example be at least 2, 3, 4, or 5 times larger than the tube-shaped body's outer diameter. The patch's diameter, in the expanded configuration, may for example range from 5mm to 30mm).

The patch, when affixed or fastened to the membrane, may be configured to withstand, e.g., until birth in case of fetoscopy, a certain range of shear forces and pressure that may be imparted in a direction normal to the surface of the patch. For example, amniotic fluid pressure range that may act or be imparted normal to the patch's surface may range from 9 mmHg at gestational stage of week 10 to 5 mmHg at 30 weeks. In TTTS, the amniotic pressure that the patch has to withstand until birth can also go up to 20 mmHg.

The figures illustrate generally, by way of example, but not by way of limitation, various embodiments of the devices and/or methods discussed in the present document.

For simplicity and clarity of illustration, elements shown in the figures have not necessarily been drawn to scale. For example, the dimensions of some of the elements may be exaggerated relative to other elements for clarity of presentation. The number of elements shown in the Figures should by no means be construed as limiting and is for illustrative purposes only. Furthermore, reference numerals may be repeated among the figures to indicate corresponding or analogous elements. References to previously presented elements are implied without necessarily further citing the drawing or description in which they appear. The figures are listed below.

**FIG. 1** is a schematic cross-sectional view of a portion of a ruptured amniotic layer sealed with a sealant, according to some embodiments;

**FIG. 2A** is a schematic assembly illustration of a membrane closure device, according to some embodiments;

**FIG. 2B** is a schematic illustration of a patch in a folded configuration located at the distal end of the membrane closure device, according to some embodiments;

**FIG. 2C** is a schematic disassembly illustration of the membrane closure device, according to some embodiments;

**FIG. 2D** is a schematic illustration of the patch in an expanded configuration after delivery through the distal end of the membrane closure device, according to some embodiments;

**FIG. 2E** is a schematic illustration of a support structure of a patch, according to an embodiment;

**FIG. 2F** is a schematic illustration a support structure of a patch, according to an alternative embodiment;

**FIG. 2G** is a schematic illustration of exemplary measures of a petal or lobe of the support structure of **FIG. 2F,** according to some embodiments;

**FIGs. 3A** and **3B** are schematic illustration showing a method of using the membrane closure device, according to some embodiments;

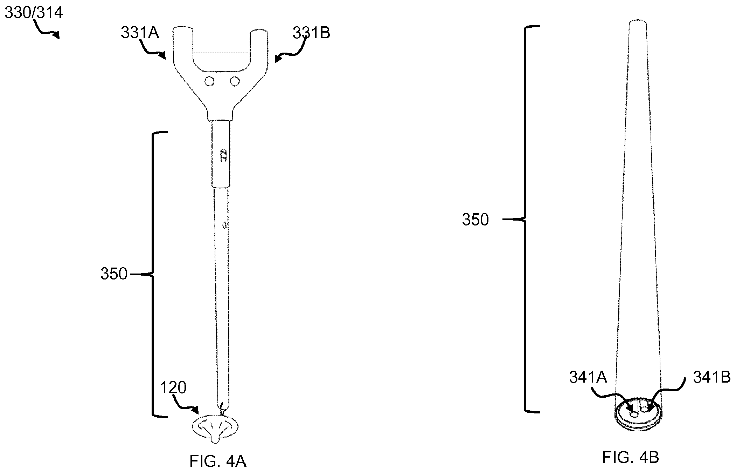

**FIG. 4A** is a schematic illustration of a fastener delivery mechanism of the membrane closure device, according to some other embodiments;

**FIG. 4B** is a schematic illustration of a distal end of the fastener delivery mechanism of **FIG. 4A;**

**FIG. 5** is a schematic illustration of a mixing chamber of the fastener delivery mechanism, according to some embodiments;

**FIGs. 6A** is a schematic illustrations showing the patch in a retained state, according to some embodiments;

**FIG. 6B** is a schematic illustration showing the patch in a released state, according to some embodiments;

**FIG. 7** is a flowchart diagram of a method for closing a perforated membrane, according to some embodiments;

**FIGs. 8A** to **8D** show images of an example implementation of a membrane closure device;

**FIGs. 9A** to **9F** show images of another example implementation of a membrane closure device;

**FIGs. 10A** to **10D** show images of exemplary in-vitro application using the membrane closure device shown in **FIGs. 8A** to **8D;** and

**FIGs. 10C** and **10D** show images of a patch with different glues applied on it.

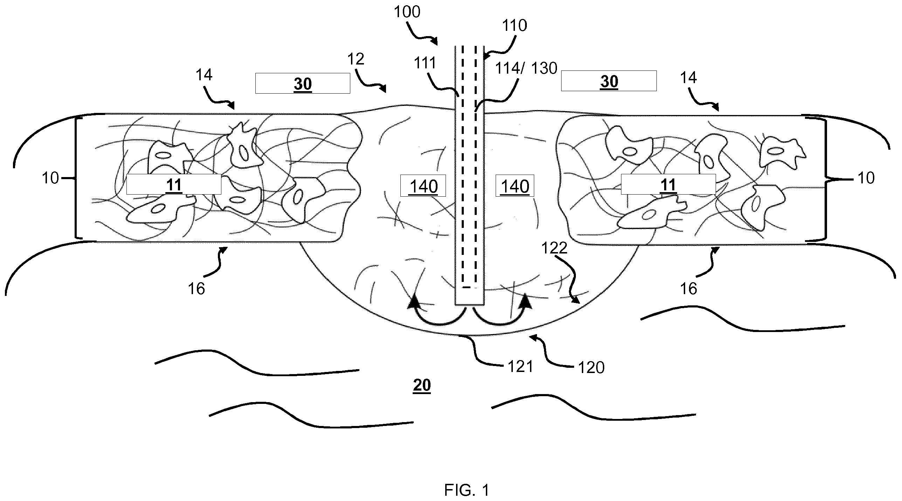

Referring to **FIG. 1,** a membrane **10** consisting of membrane tissue **11** is shown to have an inner cavity **20** for preventing fluid contained in the cavity to leak out to the membrane's surroundings **30**. Membrane **10** has a perforation site **12** extending from a proximal membrane surface **14** to an inner membrane surface **16** of membrane tissue **11**. Perforation site **12** can be sealed in a fluid-impermeable manner by employing a membrane closure device **100,** e.g., as outlined herein below. The term "fluid" as used herein may encompass liquid, gas and/or air. In some embodiments, patch **120** and/or fastener **140** may be permeable to gas and/or air, but liquid-impermeable. Optionally, patch **120** and/or fastener **140** may be semi-impermeable, e.g., permeable to allow fluid to permeate from surroundings **30** into cavity **20** but impermeable for fluid to flow from cavity **20** into surroundings **30**. Optionally, patch **120** and/or fastener **140** may be semi-impermeable to liquid and permeable to gas and/or air. Optionally, patch **120** and/or fastener **140** may be semi-impermeable to gas and/or air.

Membrane closure device **100** comprises a patch delivery mechanism **110** that comprises a tube-shaped body **111** for slidably guiding a patch **120** to perforation site **12** and, further, a fastener delivery mechanism **130** for delivering a fastener **140** to perforation site **12**. In some embodiments, lubricants (e.g., oil) may be employed for facilitating the sliding of patch **120** along the patch delivery passageway.

As exemplified in **FIG. 1,** patch **120** and fastener **140** may be employed for sealing perforation site **12**. For example, patch **120** may be arranged in an expanded configuration (e.g., expanding latitudinally) onto an inner surface **16** of membrane **10** to cover perforation site **12,** and fixed in position by a fastener **140** to seal the perforation. Otherwise stated, fastener **140** may be employed to fixedly attach or secure patch **120** in expanded configuration onto a surface of membrane **10** to seal perforation site **12**. Accordingly, fastener **140** be operative to sealantly secure patch **120** on membrane **10** to seal perforation site **12**. Generally, one or more patches may be sealantly secured onto outer surface **14** and/or inner membrane surface **16** of membrane **10**. For example, a first patch may be sealantly secured onto inner membrane surface **16** and a second patch may be sealantly secured to outer membrane surface **14**. However, merely to simplify the discussion herein, without be construed limiting, examples and/or embodiments disclosed in conjunction with the accompanying figures refer to sealantly securing patch **120** to inner membrane surface **16**.

In an embodiment, patch **120** comprises a closure sheet **121** that includes, for example, a self-growing sponge, native tissue, living tissue, Polytetrafluorethylen (PTFE), expanded Polytetrafluorethylen (ePTFE) manufactured, e.g., by employing electro-spinning; silicone-based membrane. Such silicone-based membrane may for example be coupled to structures consisting of, for example, synthetic polymer material; natural polymer material, metal; and/or any other suitable matter, composition of matter. The silicone-based membranes may be treated, e.g., using dip-coating to produce cell-instructive membranes.

Fastener **140** may comprise, for example, native tissue, a glue and/or sealant such as, for example, a biocompatible adhesive and/or a biocompatible sealant. Exemplarily, glue that may be employed can include Mussel glue, agarose glue, fibrin glue, platelet rich plasma, thrombin-containing commercial preparations, collagen, collagen slurry, gelatine sponges, matrigel and/or any other suitable matter.

Optionally, the glue and/or sealant may have a growth factor (e.g., activation with soluble epidermal growth factor (EGF) and/or insulin-link growth factor-1 (ILGF-1); and/or comprise a cell instructive scaffold like, for example, a decellularized amniotic membrane. Optionally, the cell instructive scaffold may be glued (e.g., with fibrin), and for example loaded with mixed, soluble TGF-beta or FGF-beta.

In some embodiments, fastener **140** comprises a weld produced, for example, by a laser source.

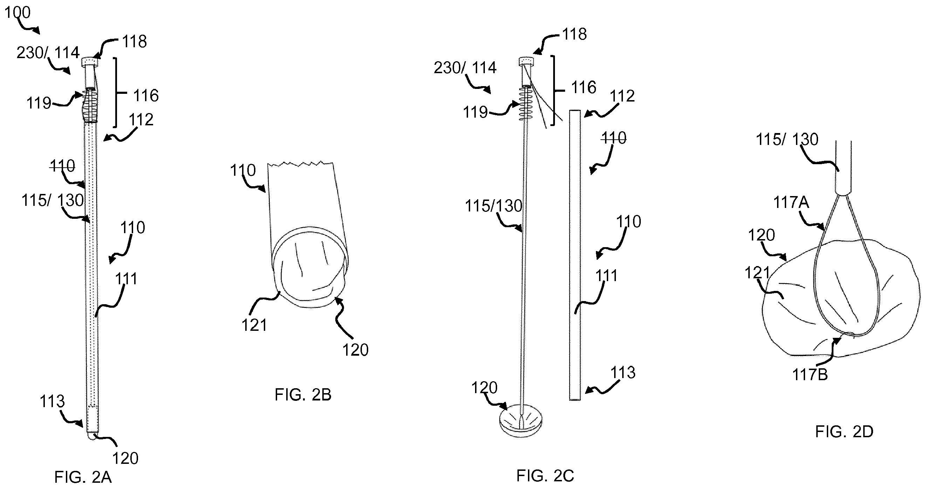

Additional reference is made to **FIGs. 2A** and **2B.** In an embodiment, patch delivery mechanism **110** comprises a tube-shaped body **111** having a proximal end **112** and a distal end **113** and defining internal to the body a patch delivery passageway. Patch delivery mechanism **110** may comprise a patch propelling device **114** that is configured to be employable by the user of membrane closure device **100** for pushing patch **120** through the patch delivery passageway from its proximal **112** to its distal end **113.**

In an embodiment, patch propelling device **114** may comprise an elongate body **115** that can be introduced into tube-shaped body **111** and which is long enough to protrude out of the distal end of tube-shaped body **111.** In an embodiment, patch propelling device **114** further comprises an actuation mechanism **116** comprising, e.g., a push button **118** and an energy storage device **119** (e.g., a mechanical energy storage device such as a compression spring) for retaining elongate body **115,** and hence patch **120,** by default in a retracted position so that patch is retained inside tube-shaped body **111.** Energy storage device **119** is arranged and configured to retain push button **118** and hence, elongate body **115** and patch **120** thereto, by default, in the retracted position. For example, energy storage device **119** may be arranged between proximal shoulders of tube-shaped body **111** and shoulders of push button **118.** Clearly, patch propelling device **114** may employ additional or alternative mechanisms for retaining patch **120** by default in the retracted position and for actuating delivery of patch **120** to perforation site **12.** Patch propelling device **114** may comprise, for example, electronic, magnetic, pneumatic, hydraulic and/or any other suitable mechanism.

When the user operably engages with the handle mechanism (e.g., manually presses onto the push button), additional energy is stored by the energy storage device, elongate body **115** advances and pushes patch **120** out of tube-shaped body **111** so that patch **120** protrudes out of the tube-shaped body's distal end **113.** While being pushed through tube-shaped body **111,** patch **120** is in a narrow configuration. Patch **120** in the narrow configuration is schematically and exemplarily shown in **FIGs. 2A** and **2B** in the patch-delivery passageway of tube-shaped body **111,** at its distal end **113.** Once patch **120** is pushed out and no longer confined within the patch delivery passageway running along tube-shaped body **111,** the patch may assume the expanded configuration. When the user then releases the pressure applied onto the push button, added energy is released causing push button to retract into its initial, default position. Clearly, patch delivery mechanism may employ additional and/or alternative handle mechanisms for manually, automatically or semi-automatically propelling patch **120** including, for example, rotary knobs, levers, and/or handles.

Additional reference is made to **FIGs. 2C** and **2D,** showing patch **120** in the expanded configuration. For instance, **FIG. 2C** shows patch **120** in the expanded configuration before being pushed into tube-shaped body **111,** and **FIG. 2D** shows a more detailed view of patch **120** shown in **FIG. 2C.** As schematically shown in **FIG. 2D,** membrane closure device **100** comprises, in an embodiment, a patch manipulation arrangement, e.g., for pulling patch **120** against inner membrane surface **16** and for preventing patch **120** from falling into cavity **20,** e.g., due to gravitation, after exiting distal end **113.** Such patch manipulation arrangement may for example comprise a patch retention element **117A** that runs within tube-shaped body **111** and terminates in a loop to form-fittingly engages with a wire loop **117B** that is affixed to closure sheet **121.** Optionally, patch retention element **117A** may be suture or wire that can be used for suturing of patch **120** to membrane **10.** In some embodiments, membrane closure device **100** may comprise a patch uncoupling mechanism (not shown) comprising, for example, a cutting element (not shown), for cutting wires **117A** and/or **117B** open, allowing both patch delivery mechanism **110** and fastener delivery mechanism **130** be removed from perforation site **12.**

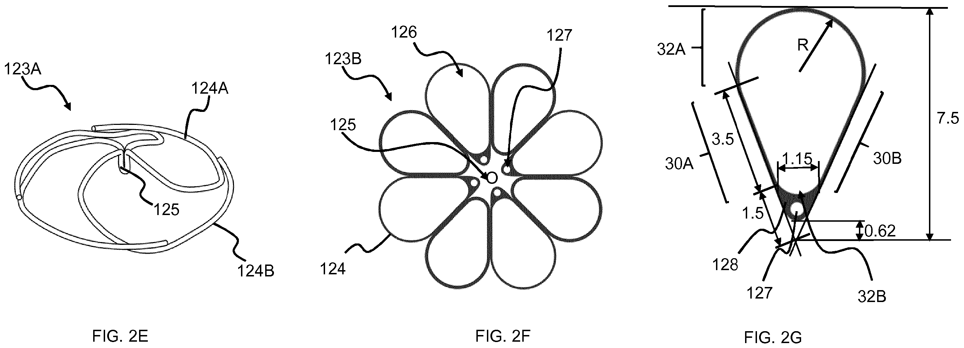

Additional reference is made to **FIGs. 2E** and **2F.** Expandable patch **120** (which may be self-expandable) further comprises a support structure **123. FIGs. 2E** and **2F** schematically show different example embodiments of such support structure **123,** respectively referenced by alphanumeric designations **"123A"** and **"123B".**

Generally, support structure **123** is configured to support closure sheet **121.** Patch **120** is an umbrella-like device that can be modified from a narrow to an expanded configuration. Optionally, support structure **123** is configured to permit patch **120** to be actuated to automatically expand from a narrow configuration to an expanded configuration. Expandable patch **120** may assume the expanded configuration after being propelled (e.g., pushed and/or pulled) through tube-shaped body **111** in the narrow configuration and after exiting distal end **113** for closing perforation site **12**

In some embodiments, support structure **123** stores more mechanical energy in the folded or narrow configuration than in the expanded configuration. Accordingly, once support structure **123** is no longer confined within the patch delivery passageway running along tube-shaped body **111,** support structure **123** may attain the expanded configuration.

In some embodiments, support structure **123** may have to be actuated to obtain the expanded configuration. For instance, support structure **123** may be made of a shape memory material such as nitinol, and can have a low temperature martensite state, with a martensite transition temperature below body temperature, and a comparatively high temperature austenite state at or above body temperature. Below body temperature, support structure **123** may be in its martensitic state, which is relatively soft, plastic, and easy to shape. In its austenite state, support structure **123** may assume a memorized harder material state in which support structure **123** is more difficult to deform than in the martensitic state. In some embodiments, in order for patch **120** to automatically obtain the desired shape in the expanded configuration, the materials employed for producing support structure **123** may be subjected to temperature-based shape-setting. Heating of closure sheet **121** to above body temperature where it may assume its austenite state with its memorized harder material state may in some embodiments be employed including, for example, electric charge/current flowing via a wire/electric-circuit (not shown) (possibly made of copper) or by other means such as laser light propagating via tube-shaped body **111.**

According to some embodiment, support structure **123,** when in the expanded configuration, may be resistant to bending forces of, for example, at least 1 Newton, 2 Newton, 5 Newton, or at least 10 Newton.

During the transition from the narrow to the expanded configuration, the support structure **123** urges closure sheet **121** to unfold.

In an embodiment, closure sheet **121** may be affixed to support structure **123.** Optionally, support structure **123** may be embedded (partially or fully) in closure sheet **121,** glued to closure sheet **121,** and/or otherwise affixed to closure sheet **121.** Optionally, in the expanded configuration, closure sheet **121** may be stretchably coupled to support structure **123.**

In an embodiment, support structure **123** comprises struts that may for example be made of wires or coils. As schematically shown in **FIG. 2E,** support structure **123A** may for example comprise at least two wires **124A** and **124B** that assume, in the expanded configuration, an S-shaped form. In the expanded configuration, the at least two S-shaped wires **124A** and **124B** may be rotated relative to each other at their midpoints **125** such that the arcuate portions of the S-shaped wires complement a circular-like strut portion running along the outer edge of closure sheet **121.** Optionally, a support structure 123 may comprise wires that assume in the expanded configuration, T-shaped, U-shaped, C-shaped, longitudinal wires and/or any other desired form.

As schematically shown in **FIG. 2F,** support structure **123B** may comprise wires delineating in the expanded configuration the shape of a plurality of petals or lobes **126.**

Generally, a petal may assume, in the extended configuration, any polygonal shape and therefore have curved and/or straight vertices and/or edges. For example, a petal **126** can have a triangular or substantially triangular shape.

In an embodiment, a wire of support structure **123B** may delineate a curve resembling the shape of petalled flower or have a "Shamrock-like" shape. The curve delineated by support structure **123B** may exemplarily be termed "rhodonea" or "rose curve" and may be constructed using, for example, one of the following polar equations (1) or (2):

$$r = a\ sin(n\vartheta) \qquad\qquad (1)$$

or

$$r = a\ cos(n\vartheta) \qquad\qquad (2)$$

As shown exemplarily in **FIG. 2F,** $n=4$, resulting in that the "rose" is 8-petalled. It should be noted that additional or alternative approaches or mathematical formulas can be employed to arrive at a support structure **123B** delineating, e.g., a petalled contour or curve as exemplarily shown in **FIG. 2F,** and that the number of petals or lobes shown in **FIG. 2F** should by no means be construed in a limiting manner. Optionally, a petal or lobe-shaped geometry having the form of a petal **126** may be (virtually) rotated around midpoint **125** for constructing a petalled flower structure.

Further reference is made to **FIG. 2G.** A lobe **126,** which may be made of a wire, may have the measures (in millimeters) as exemplarily indicated in **FIG. 2G.**

Each lobe **126** may comprise two radial portions **30A** and **30B** virtually originating from midpoint **125** and terminating in a distal convex curve **32A** having a radius **R.** Optionally, a lobe **126** may comprise a convex curve **32B** that is proximal to midpoint **125.** Optionally, two neighboring lobes **126** may be coupled with each other at adjacent radial portions **30.** Optionally, adjacent radiation portions **30** of two neighboring lobes **126** may abut against each other, yet be detached from one another.

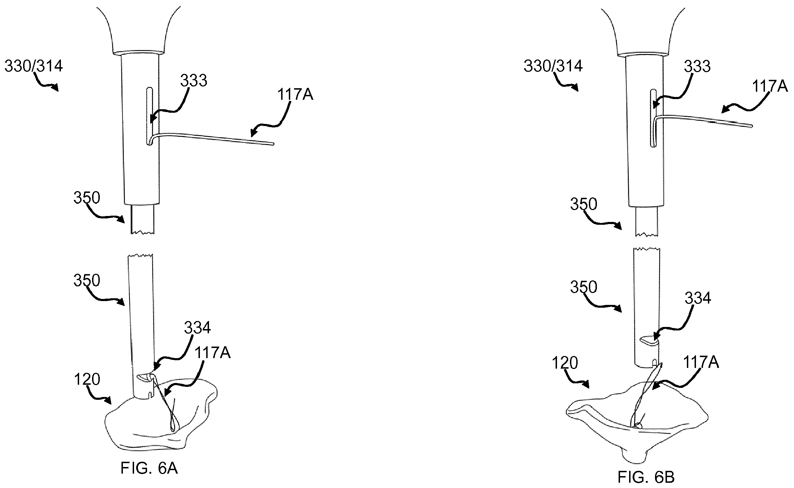

In an embodiment, at least one lobe **126** of support structure **123B** may comprise coupling elements **127,** e.g., for coupling support structure **123B** with patch retention element **117A,** which is exemplified and schematically illustrated in **FIGs. 6A** and **6B.** In an embodiment, coupling element **127** may be located proximal to geometric midpoint **125** of support structure **123B.** Optionally, lobe **126** may comprise a base **128** that is proximal to midpoint **125.** Optionally, base **128** may comprise coupling element **127.**

Optionally, two coupling elements **127** of respective lobes **126** may be located opposite each other. Optionally, a coupling element **127** may comprise an eyelet, a hook and/or any other suitable configuration to enable the coupling of support structure **123B,** and thus of patch **120,** with elongate body **115** of patch propelling device **114.**

Optionally, two lobes **126** that are located opposite each other may each include a coupling element **127.** In embodiments where the number of lobes **126** is even, only every second lobes **126** may comprise a coupling element **127.** In some embodiments, lobes **126** may have a substantially symmetrical geometry, e.g., as shown in **FIGs. 2F** and

2G. In some other embodiments, lobes **126** may have a non-symmetrical geometry (not shown).

In some embodiments, support structure **123** may be manufactured, for example, from a sheet of shape memory alloy (e.g., Nitinol) by employing laser-cutting technologies, and/or by welding and/or braiding of wires. Support structure **123** may be, in some embodiments, basket-like shaped.

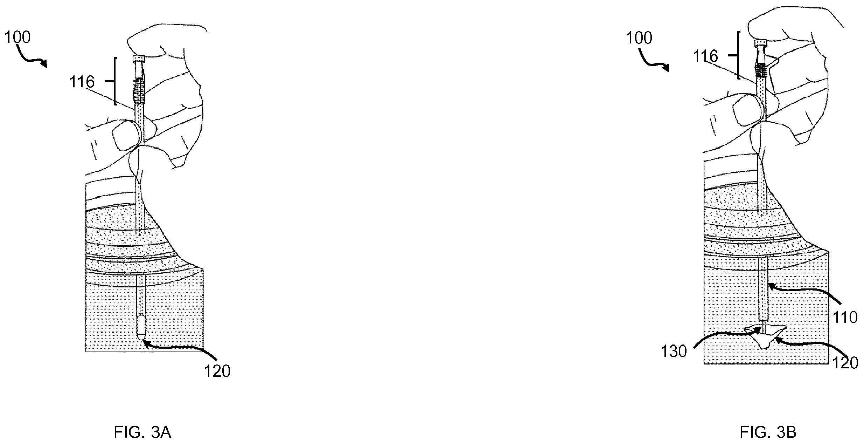

Additional reference is made to **FIGs. 3A** and **3B,** which schematically show a method of using the membrane closure device, according to some embodiments. More specifically, an exemplary in-vitro deployment of patch **120** by the user is schematically illustrated. **FIG. 3A** shows patch **120** in tube-shaped body **111** in the folded configuration, and **FIG. 3B** shows patch **120** in the expanded configuration.

According to some embodiments, patch **120** may have, in the expanded configuration, a concave surface **122** facing inner membrane surface **16.** The patch's concave surface **122** may facilitate the gathering or holding of glue and/or sealant **140** by closure sheet **121,** as is shown schematically in **FIG. 1,** and prevent the glue and/or sealant **140** to spill into cavity **20.** Optionally, patch **120** may be pulled or held against inner membrane surface **16** circumferencing perforation site **12** for up to 5, 10, 20, 30 or 40 seconds to ensure that the patch is sealantly secured to membrane **10.** After patch **120** is secured so that perforation site **12** is sealantly lidded, patch retention element **117A** may be uncoupled (e.g., cut or released) by operably employing the patch uncoupling mechanism (not shown). Patch retention element **117A** and wire **117B** can be biocompatible and, optionally, biodegradable and/or bioabsorbable.

As already briefly outlined herein, membrane closure device **100** comprises a fastener delivery mechanism **130** for delivering a fastener **140** to perforation site **12.** The fastener delivery mechanism may be manually, automatically or semi-automatically operable.

In some embodiments, a fastener delivery mechanism may be a device that is separate and independently operable from a patch propelling device. In some other embodiments, a fastener delivery mechanism may be integral to a patch propelling device. Although the discussion that follows with respect to the accompanying figures refers to embodiments where a fastener delivery mechanism is integral to a patch propelling device, this should by no means be construed limiting.

According to some embodiments, fastener delivery mechanism **130** is configured to enable controllably delivering glue **140,** e.g., once patch **120** is deployed at perforation site **12** and, optionally, after it assumed the expanded configuration, for securing patch **120** with the delivered glue **140** to membrane **10.** For example, fastener delivery mechanism **130** may comprise a glue injection mechanism (not shown). For example, glue injection mechanism may comprise a cavity defining internal to elongate body **115** a fastener-delivery passageway that is configured to receive glue **140.** In an embodiment, the glue injection mechanism may further comprise a plunger (not shown) that is provided on the front end thereof with a piston that is slidably movable within the cavities of elongate body **115** for pressing glue **140** out of the fastener-delivery passageway.

Additional reference is made to **FIGs**. **4A and 4B.** In some embodiments, a fastener-delivery mechanism **330** may have a Y-shaped or bifurcated configuration and may comprise at its bifurcated distal end portions a plurality of barrels (e.g., barrels **331A** and **331B**) of a glue injection mechanism (not shown). The plurality of barrels may converge to a shared elongate body portion **350** where they are in separate fluid communication with a plurality of fastener-delivery passageways (e.g., passageways **341A** and **341B)** running along elongate body portion **350** for the separate delivery and, optionally, mixing, of a corresponding plurality of glue compositions of matters (e.g., two glue components). The glue injection mechanism can for example comprise, analogous to the embodiments discussed in **FIGs. 2A** and **2B,** a plurality of plungers (not shown) that are each provided on the front end thereof with a piston (not shown).The piston (not shown) can be slidably movable within the cavities of elongate body of barrels **331A** and **331B** for pressing glue **140** out of the fastener-delivery passageways.

In some embodiments, fastener delivery mechanism **330** may also function as a patch propelling device **314.** Patch propelling device **314** may for example be slidably insertable into tube-shaped body **110** for pushing patch **120** outside the distal end of tube-shaped body **111.**

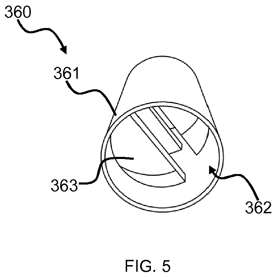

Further reference is made to **FIG. 5.** In some embodiments, fastener delivery mechanism **330** may comprise a mixer **360** operative to mix two or more adhesive-components with each other for producing an adhesive. In some embodiments, elongate body **350** may terminate in mixer **360.** Optionally, mixer **360** may be implemented as a static or dynamic mixer.

Mixer **360** may for example comprise a mixing body **361** defining therein a mixing cavity **362.** Fins **363** may protrude inwardly into mixing cavity **362** for the mixing of different adhesive matter of composition. In an embodiment, fins **363** are alternatingly arranged opposite each other along the longitudinal axis of mixing body **361.** In an embodiment, fins **363** are arranged in a screw-like progression along the tube-shaped mixing body.

Additional reference is made to **FIGs. 6A** and **6B.** As already briefly outlined herein, the patch manipulation arrangement of membrane closure device **100** may for example comprise a patch retention element **117A** that runs within tube-shaped body **111** and terminates at its distal end that is releasably coupled with patch **120.** In one embodiment, patch retention element **117A** may be rod (not shown) that form-fittingly engages with a looped wire or thread **117B** that is affixed to patch **120,** like a latch or plunger. In another embodiment, patch retention element **117A** may comprise a wire or thread that runs within elongate body portion **350** and exit therefrom via a distal slit **334** for holding patch **120,** and may further comprise a rod that is releasably coupled with a looped portion of the wire.

Either way, a proximal end of patch retention element **117A** may protrude from fastener-delivery passageway, e.g., via a proximal slit **333,** to allow the user to hold it for manipulation of the distal end of patch retention element **117A.** FIG. 6A schematically shows patch **120** in a retained state, e.g., attached to patch retention element **117A.** FIG. 6B schematically shows patch **120** in an uncoupled or released state from patch retention element **117A.** According to the above noted first embodiment, patch **120** may be released by lifting the rod, exemplarily causing the thread to uncouple from elongate body portion **350.** Optionally, the thread may be released from a form-fitting and/or frictional coupling state from the distal end of elongate body portion **350.**

In another embodiment, patch **120** may be coupled to the distal part of elongate portion **350** of fastener delivery mechanism **130.** Optionally, elongate portion 350 may be slidable from a retracted to an extended position, e.g., by lifting or lowering of a handle portion of patch retention element **117A.** The handle portion may be the portion of patch retention element **117A** that protrudes out of proximal slit **333.**

Embodiments disclosed herein may be combined with additional wound closure techniques including, for example, cross-linking technologies and/or membrane healing through biological stimulation.

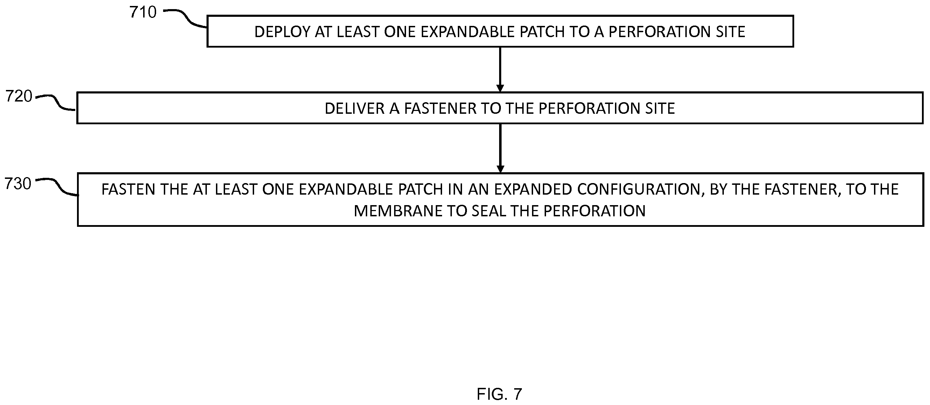

Further reference is now made to **FIG. 7.** As indicated by step **710,** a method for closing a perforated membrane comprises, in an embodiment, deploy at least one expandable patch to a perforation site. Optionally, deploying the at least one expandable patch may include pulling or pushing the patch against the membrane surface to which the patch shall be affixed. For example, referring to **FIG.1,** patch **120** may be pulled in proximal direction towards the inner surface.

As indicated by step **720,** the method may further include deliver a fastener to the perforation site. As indicated by step **730,** the method may include fastening the at least one expandable patch in an expanded configuration, by the fastener, to the membrane to seal the perforation.

EXAMPLES:

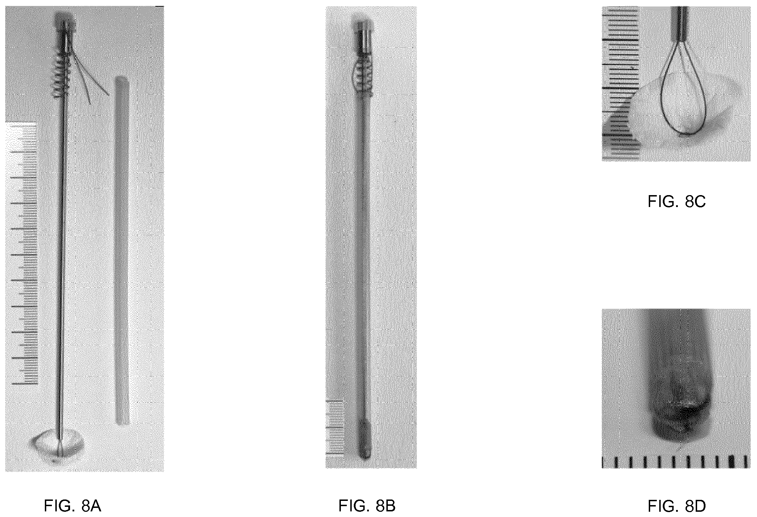

**FIGs. 8A to 8D** exemplarily show images of an example implementation of a membrane closure device. More specifically, FIG. **8A** exemplarily shows an image of the elongate body or pushing cylinder of a patch propelling device responsible of pushing the patch out and retaining it for its positioning onto the uterine wall. **FIG. 8B** exemplarily shows an image of the membrane closure with the patch loaded inside thereof. **FIG. 8C** is an image exemplarily showing a more detailed view of the pushing, inner cylinder with the wire retaining the patch when deployed and an inner compartment for glue injection. **FIG. 8D** exemplarily shows that the transparent appearance of the patch in the folded state is due to the employment of oil that was necessary to load the patch into the tube.

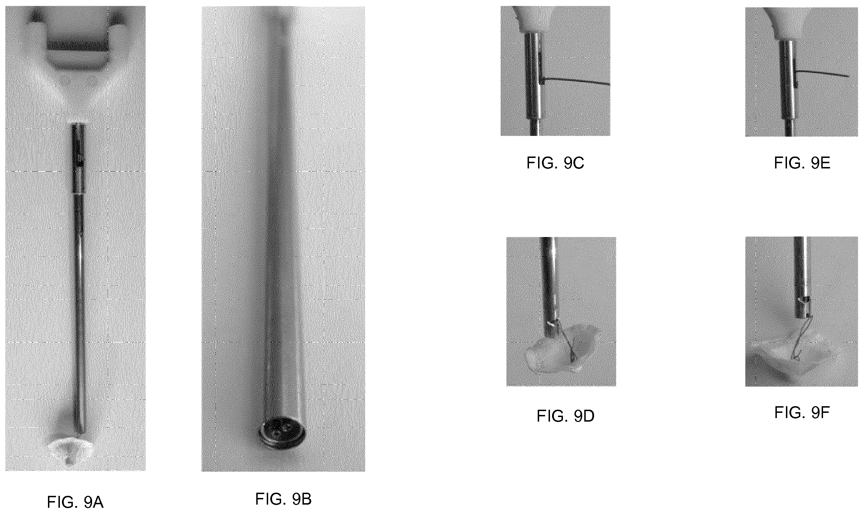

**FIGs. 9A to 9F** show images of another example implementation of a membrane closure device. More specifically, **FIG. 9A** shows an image of an exemplary inner cylinder responsible for pushing the patch out and retaining it for its positioning onto the uterine wall. **FIG. 9B** is an image exemplarily showing an image of two smaller tubes that extend throughout the whole construct of the pushing cylinder to deliver a sealant composed of two components. **FIGs. 9C** and **9D** are image exemplarily showing the membrane closure device with the patch attached to it via a suture wire loop and retained by a wire controlled at the top here in its closed state. **FIGs. 9E-9F** exemplarily shows this same wire in its open state (patch retention element **117A** is uncoupled from wire loop **117B**) for releasing the patch.

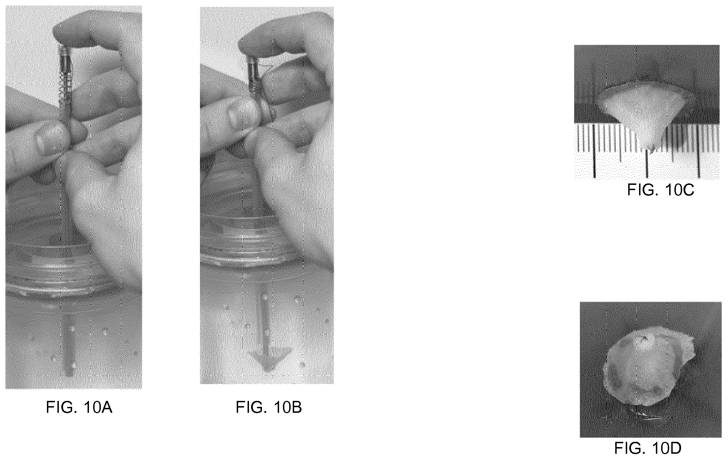

**FIGs. 10A** and **10B** show images of exemplary in-vitro application of the membrane closure device shown in **FIGs. 8A to 8D.** More specifically, **FIGs. 10A** and **10B** show the deployment of the patch in a wet container.

**FIG. 10C** and **10D** show different views of the patch affixed to a wet surface with Agarose glue.

The membrane closure devices and components shown in **FIGs. 8A to 10D** fitted perfectly to the instruments used in clinics for fetoscopy as tested directly on catheters provided by the obstetric department. A metallic mold for strict reproducible receptor fabrication was produced and a series of umbrellas were electrospun. Their compatibility to the membrane closure devices was verified by simple insertion into it and its deployment tested in water. A test of feasibility included injecting fibrin glue through the two-chamber syringe into the concave surface of the patch. The reception capacity was confirmed by this experiment. A nitinol umbrella backbone was manufactured by MeKo™ . The nitinol umbrella backbone was embedded into a silicone sheet (the closure sheet). Optionally, the nitinol umbrella backbone could be coated by the electro-spun membrane.

Additional examples:

Example 1 includes a membrane closure device for closing a perforation in a membrane, comprising: a patch delivery mechanism for guiding at least one expandable patch to a perforation site of a membrane; and a fastener delivery mechanism for delivering a fastener to the perforation site and for fastening the at least one expandable patch by the fastener to the membrane to seal the perforation in the membrane by the at least one expandable patch.

Example 2 includes the subject matter of example 1 and, optionally, at least one tube or tube-shaped body having a distal and a proximal end for delivering the at least one expandable patch in a folded configuration via the tube from the distal to the proximal end for attaching the at least one expandable patch in an expanded configuration to an inner surface of the membrane.

Example 3 includes the subject matter of example 1 or 2 and, optionally, wherein the at least one expandable patch is a self-expandable patch.

Example 4 includes the subject matter of example 3 and, optionally, wherein the self-expandable patch is an umbrella-like device comprising struts and a closure sheet affixed to the struts.

Example 5 includes the subject matter of example 4 and, optionally, wherein the struts comprise at least two S-shaped wires.

Example 6 includes the subject matter of example 5 and, optionally, wherein the struts comprise shape-memory material composed of, for example, Nitinol.

Example 7 includes the subject matter of any one of examples 1 to 6 and, optionally, wherein the at least one expandable patch, in the expanded configuration, has a concave surface facing the membrane for the reception of glue.

Example 8 includes the subject matter of any one of examples 2 to 7 and, optionally, wherein the fastener delivery mechanism comprises the at least one tube for delivering the fastener to the perforation site.

Example 9 includes the subject matter of any one of the examples 2 to 7 and, optionally, wherein the fastener delivery mechanism comprises at least one other tube, different from the at least one tube, for delivering the fastener to the perforation site.

Example 10 includes the subject matter of any one of the examples 1 to 9 and, optionally, wherein the fastener comprises any one of the following: biocompatible adhesive, biocompatible sealant, sealant-inducing material, and/or weld.

Example 11 includes the subject matter of any one of the examples 1 to 10 and, optionally, wherein the fastener delivery mechanism comprises an energy source such as, for example, a laser source.

Example 12 includes the subject matter of any one of the examples 1 to 11 and, optionally, wherein the fastener delivery mechanism comprises an adhesive mixing chamber.

Example 13 includes the subject matter of example 12 and, optionally, wherein the adhesive mixing chamber comprises a tube-shaped body and fins, wherein the fins protrude inwardly into the tube-shaped body for the mixing of different adhesive matter of composition.

Example 14 includes the subject matter of example 13 and, optionally, wherein the fins are alternatingly arranged opposite each other along the tube's longitudinal axis.

Example 15 includes the subject matter of examples 13 or 14 and, optionally, wherein the fins are arranged in a screw-like progression along the tube-shaped body.

Example 16 includes the subject matter of any one of the examples 1 to 15 and, optionally, comprising a patch uncoupling mechanism for releasing or cutting the at least one expandable patch from the membrane closure device following fastening of the at least one expandable patch to the inner membrane surface of the perforation site of the membrane.

Example 17 includes the subject matter of any one of the examples 1 to 16 and, optionally, wherein the fastener comprises any one of the following: a growth factor and/or a cell instructive scaffold like, for example, a decellularized amniotic membrane.

Example 18 includes the membrane closure device according to any of the example 1 to 17 for use in treating a perforation of a fetal membrane, for use in treating a perforation of a vascular membrane, for use in treating a perforation of a cardiovascular membrane and/or for use in treating a tympanic membrane.

Example 19 includes a method for closing a perforation in a membrane by a membrane closure device, comprising:

delivering at least one expandable patch to a perforation site; delivering a fastener to the perforation site; and fastening the at least one expandable patch by the fastener in an expanded configuration to the membrane to seal the perforation.

Example 20 includes the subject matter of example 19 and, optionally, operably deploying a plurality of patches.

Unless otherwise stated, the use of the expression "and/or" between the last two members of a list of options for selection indicates that a selection of one or more of the listed options is appropriate and may be made.

In the claims or specification of the present application, unless otherwise stated, adjectives such as "substantially" and "about" modifying a condition or relationship characteristic of a feature or features of an embodiment of the invention, are understood to mean that the condition or characteristic is defined within tolerances that are acceptable for operation of the embodiment for an application for which it is intended.

It should be understood that where the claims or specification refer to "a" or "an" element and/or feature, such reference is not to be construed as there being only one of that element and/or feature.

Terms used in the singular shall also include the plural, except where expressly otherwise stated or where the context otherwise requires.

In the description and claims of the present application, each of the verbs, "comprise" "include" and "have", and conjugates thereof, are used to indicate that the object or objects of the verb are not necessarily a complete listing of components, elements or parts of the subject or subjects of the verb.

The various features and steps discussed above can be mixed and matched by one of ordinary skill in the art in accordance with principles described herein.

Although the disclosure has been provided in the context of certain embodiments and examples, it will be understood by those skilled in the art that the disclosure extends beyond the specifically described embodiments to other alternative embodiments and/or uses and obvious modifications and equivalents thereof. Accordingly, the disclosure is not intended to be limited by the specific disclosures of embodiments herein.

In the description, unless otherwise stated, adjectives such as "substantially" and "about" that modify a condition or relationship characteristic of a feature or features of an embodiment of the invention, are to be understood to mean that the condition or characteristic is defined within tolerances that are acceptable for operation of the embodiment for an application for which it is intended.

It is appreciated that certain features of the invention, which are, for clarity, described in the context of separate embodiments or examples, may also be provided in combination in a single embodiment. Conversely, various features of the invention, which are, for brevity, described in the context of a single embodiment, may also be provided separately or in any suitable sub-combination or, as suitable, in any other described embodiment of the invention. Certain features described in the context of various embodiments are not to be considered essential features of those embodiments, unless the embodiment is inoperative without those elements.

It is important to note that methods discussed herein are not limited to those diagrams or to the corresponding descriptions. For example, the methods may include additional or even fewer processes or operations in comparison to what is described in the figures. In addition, embodiments of the method are not necessarily limited to the chronological order as illustrated and described herein.

It is noted that the term "exemplary" is used herein to refer to examples of embodiments and/or implementations, and is not meant to necessarily convey a more-desirable use-case.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

While the invention has been described with respect to a limited number of embodiments, these should not be construed as limitations on the scope of the invention, but rather as exemplifications of some of the embodiments.

**Claims**

1. A membrane closure device (100) for closing a perforation in a membrane (10), comprising:

   a patch delivery mechanism (110) for guiding at least one expandable patch (120) to a perforation site (12) of a membrane (10); and
   a fastener delivery mechanism (130, 330) for delivering a fastener (140) to the perforation site (12) and for fastening the at least one expandable patch (120) by the fastener (140) to the membrane (10) to seal the perforation in the membrane (10) by the at least one expandable patch (120).

2. The membrane closure device (100) according to claim 1, comprising at least one tube having a distal and a proximal end for delivering the at least one expandable patch (120) in a folded configuration via the tube from the distal to the proximal end for attaching the at least one expandable patch (120) in an expanded configuration to an inner surface (16) of the membrane (10).

3. The membrane closure device (100) according to claim 1 or claim 2, wherein the at least one expandable patch (120) is a self-expandable patch (120).

4. The membrane closure device (100) according to claim 3, wherein the self-expandable patch (120) is an umbrella-like device comprising a support structure (123A, 123B) including struts and a closure sheet (121) affixed to the struts.

5. The membrane closure device (100) according to claim 4, wherein the struts comprise at least two wires (124A, 124B) that assume, in the expanded configuration, an S-shaped form or the form or contour of a petalled flower.

6. The membrane closure device (100) according to claim 5, wherein the struts comprise shape-memory material composed of, for example, Nitinol.

7. The membrane closure device (100) according to any of the preceding claims, wherein the at least one expandable patch (120), in the expanded configuration, has a concave surface (122) facing the membrane (10) to facilitate the reception of glue.

8. The membrane closure device (100) according to any of the preceding claims, wherein the fastener (140) comprises any one of the following: biocompatible adhesive, biocompatible sealant, sealant-inducing material, a healing-inducing material, a plug, and/or weld.

9. The membrane closure device (100) according to any of the preceding claims, wherein the fastener delivery mechanism (130, 330) comprises a mixer (360) that is included in the tube-shaped body for the mixing of components to produce an adhesive.

10. The membrane closure device (100) according to claim 9, wherein the mixer (360) comprises a mixing body (361) and fins (363), wherein the fins (363) protrude inwardly into the mixing body (361) for the mixing of different adhesive matter of composition.

11. The membrane closure device (100) according to any of the preceding claims, comprising a patch uncoupling mechanism for releasing the at least one expandable patch (120) from the membrane closure device following fastening of the at least one expandable patch (120) to the inner membrane surface (16) of the perforation site (12) of the membrane (10).

12. The membrane closure device (100) according to any of the preceding claims, wherein the fastener (140) comprises any one of the following: a growth factor and/or a cell instructive scaffold like, for example, a decellularized amniotic membrane.

13. The membrane closure device (100) according to any of the preceding claims for use in treating a perforation of a fetal membrane, for use in treating a perforation of a vascular membrane, for use in treating a perforation of a cardiovascular membrane and/or for use in treating a tympanic membrane.

14. A method for closing a perforation in a membrane (10) by a membrane closure device (100), comprising:

   delivering at least one expandable patch (120) to a perforation site;
   delivering a fastener (140) to the perforation site; and
   fastening the at least one expandable patch (120) by the fastener (140) in an expanded configuration to the membrane (10) to seal the perforation.

15. The method of claim 14, comprising operably deploying a plurality of patches (120).

FIG. 1

FIG. 2A

FIG. 2B

FIG. 2C

FIG. 2D

13

FIG. 2E

FIG. 2F

FIG. 2G

EP 3 300 669 A1

FIG. 3A

FIG. 3B

FIG. 4A

FIG. 4B

EP 3 300 669 A1

FIG. 5

FIG. 6B

FIG. 6A

710 — DEPLOY AT LEAST ONE EXPANDABLE PATCH TO A PERFORATION SITE

720 — DELIVER A FASTENER TO THE PERFORATION SITE

730 — FASTEN THE AT LEAST ONE EXPANDABLE PATCH IN AN EXPANDED CONFIGURATION, BY THE FASTENER, TO THE MEMBRANE TO SEAL THE PERFORATION

FIG. 7

FIG. 8C

FIG. 8D

FIG. 8B

FIG. 8A

FIG. 9E

FIG. 9F

FIG. 9C

FIG. 9D

FIG. 9B

FIG. 9A

FIG. 10C

FIG. 10D

FIG. 10A

FIG. 10B

Europäisches
Patentamt

European
Patent Office

Office européen
des brevets

**EUROPEAN SEARCH REPORT**

Application Number

EP 17 15 4743

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 353 513 A1 (TYCO HEALTHCARE [US]) 10 August 2011 (2011-08-10) * paragraphs [0027], [0028], [0047]; figures 10-14 * | 1,2,11,13 | INV. A61B17/00 |
| X | US 2008/058710 A1 (WILK PETER J [US]) 6 March 2008 (2008-03-06) * paragraph [0014]; figures 3,6B,7 * | 1,2,11,13 | |
| X | WO 2013/009872 A1 (UNIV MICHIGAN [US]; ORAL HAKAN [US]; MORADY FRED [US]) 17 January 2013 (2013-01-17) * figures 3A,6C * | 1,2,11,13 | |
| X | WO 2011/137224 A1 (SHERWINTER DANNY AZRIEL [US]; SIGMON JOHN CROWDER [US]; SURTI VIHAR C) 3 November 2011 (2011-11-03) * paragraphs [0030], [0034]; figures 6,8,10 * | 1,2,11,13 | |
| X | EP 1 595 504 A1 (MEDTRONIC VASCULAR INC [US]) 16 November 2005 (2005-11-16) * figures 4,5 * | 1,2,11,13 | TECHNICAL FIELDS SEARCHED (IPC) A61B |
| X | WO 2011/057282 A2 (CARDIOVASCULAR TECHNOLOGIES INC [US]; HOUSER RUSSELL A [US]; BOWER JOH) 12 May 2011 (2011-05-12) * paragraph [0233]; figures 64A,64B,81E * | 1,2,11,13 | |
| X | DE 20 2010 011724 U1 (OSYPKA AG [DE]) 4 November 2010 (2010-11-04) * figures 7-9 * | 1,2,11,13 | |
| X | US 2010/191295 A1 (TRIEU HAI H [US] ET AL) 29 July 2010 (2010-07-29) * figures 6A,6B,6D * | 1,2,11,13 | |

-/--

~~The present search report has been drawn up for all claims~~

| Place of search | Date of completion of the search | Examiner |
|---|---|---|
| Munich | 7 August 2017 | Assion, Jean-Charles |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

**EUROPEAN SEARCH REPORT**

Application Number

EP 17 15 4743

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2014/150154 A1 (ESSENTIAL MEDICAL INC [US]) 25 September 2014 (2014-09-25) * paragraphs [0150], [0151]; figures 3G,25,34 * ----- | 1,2,11, 13 | |

TECHNICAL FIELDS
SEARCHED (IPC)

~~The present search report has been drawn up for all claims~~

| Place of search | Date of completion of the search | Examiner |
|---|---|---|
| Munich | 7 August 2017 | Assion, Jean-Charles |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

Application Number

EP 17 15 4743

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1, 2, 11, 13-15

☐ The present supplementary European search report has been drawn up for those parts of the European patent application which relate to the invention first mentioned in the claims (Rule 164 (1) EPC).

Europäisches
Patentamt
European
Patent Office
Office européen
des brevets

**LACK OF UNITY OF INVENTION
SHEET B**

Application Number

EP 17 15 4743

The Search Division considers that the present European patent application does not comply with the requirements of unity of invention and relates to several inventions or groups of inventions, namely:

1. claims: 1, 2, 11, 13-15

        membrane closure device with a tube and a patch uncoupling
        mechanism
                        ---

2. claims: 1, 3-7, 13

        membrane closure device with a patch
                        ---

3. claims: 1, 8, 12, 13

        membrane closure device with a fastener
                        ---

4. claims: 1, 9, 10, 13

        membrane closure device with a mixer
                        ---

## ANNEX TO THE EUROPEAN SEARCH REPORT
## ON EUROPEAN PATENT APPLICATION NO.

EP 17 15 4743

This annex lists the patent family members relating to the patent documents cited in the above-mentioned European search report.
The members are as contained in the European Patent Office EDP file on
The European Patent Office is in no way liable for these particulars which are merely given for the purpose of information.

07-08-2017

| Patent document cited in search report | | Publication date | Patent family member(s) | | Publication date |
|---|---|---|---|---|---|
| EP 2353513 | A1 | 10-08-2011 | AU 2011200315 A1<br>CA 2728784 A1<br>EP 2353513 A1<br>JP 2011161231 A<br>US 2011196420 A1 | | 25-08-2011<br>08-08-2011<br>10-08-2011<br>25-08-2011<br>11-08-2011 |
| US 2008058710 | A1 | 06-03-2008 | NONE | | |
| WO 2013009872 | A1 | 17-01-2013 | EP 2731493 A1<br>US 2013018413 A1<br>WO 2013009872 A1 | | 21-05-2014<br>17-01-2013<br>17-01-2013 |
| WO 2011137224 | A1 | 03-11-2011 | EP 2563231 A1<br>US 2012016409 A1<br>US 2016228110 A1<br>WO 2011137224 A1 | | 06-03-2013<br>19-01-2012<br>11-08-2016<br>03-11-2011 |
| EP 1595504 | A1 | 16-11-2005 | EP 1595504 A1<br>JP 2005324019 A<br>US 2005256532 A1 | | 16-11-2005<br>24-11-2005<br>17-11-2005 |
| WO 2011057282 | A2 | 12-05-2011 | CN 102711631 A<br>EP 2498689 A2<br>JP 5626738 B2<br>JP 2013509974 A<br>KR 20120101677 A<br>WO 2011057282 A2 | | 03-10-2012<br>19-09-2012<br>19-11-2014<br>21-03-2013<br>14-09-2012<br>12-05-2011 |
| DE 202010011724 | U1 | 04-11-2010 | NONE | | |
| US 2010191295 | A1 | 29-07-2010 | NONE | | |
| WO 2014150154 | A1 | 25-09-2014 | US 2016007977 A1<br>WO 2014150154 A1 | | 14-01-2016<br>25-09-2014 |

EPO FORM P0459

For more details about this annex : see Official Journal of the European Patent Office, No. 12/82

**EP 3 300 669 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LEE et al.** *Nature,* 2007, vol. 448, 338-41
- **BILIC et al.** *American Journal of Obstetrics and Gynecology,* 2010, vol. 202 (85), e1-9
- **HALLER et al.** *Acta biomaterialia,* 2012, vol. 8, 4365-70